# EUROPEAN PATENT APPLICATION

(11) **EP 4 450 972 A1**
(43) Date of publication of application: **23.10.2024**
(21) Application number: 23382377.2
(22) Date of filing: 21.04.2023
(51) Int. Cl.: G01N 33/574, C12Q 1/6886

(54) **IN VITRO METHOD FOR PREDICTING CANCER PATIENT RESPONSE TO PD-1 AND/OR PD-L1 INHIBITORS**

(71) Applicant: Fundación Instituto de Investigación Sanitaria de Santiago de Compostela, 15706 Santiago de Compostela, A Coruña (ES); Servizo Galego de Saude, 15703 Santiago de Compostela (ES); Universidade de Santiago de Compostela, 15782 Santiago de Compostela (ES); Roche Farma, S.A.U., 28042 Madrid (ES); Consorcio Centro de Investigación Biomédica en Red, 28029 Madrid (ES)
(72) Inventor: DÁVILA IBÁÑEZ, Ana Belén, 15706 Santiago de Compostela (ES); LÓPEZ LÓPEZ, Rafael, 15706 Santiago de Compostela (ES); DÍAZ PEÑA, Roberto, 15706 Santiago de Compostela (ES); BRAVO LÓPEZ, Susana, 15706 Santiago de Compostela (ES); MONDELO MACÍA, Patricia, 15706 Santiago de Compostela (ES); MUINELO ROMAY, Laura, 15706 Santiago de Compostela (ES); LEÓN MATEOS, Luis Ángel, 15706 Santiago de Compostela (ES); GARCÍA GONZÁLEZ, Jorge José, 15706 Santiago de Compostela (ES)
(74) Representative: Hoffmann Eitle

(57) **Abstract**

The present invention refers to the medical field. Particularly, it refers to an *in vitro* method for predicting the response of patients suffering from cancer to a treatment with PD-1 and/or PDL1 inhibitors, and to an *in vitro* method for recommending whether to treat patients suffering from cancer with PD-1 and/or PDL1 inhibitors, or for classifying patients suffering from cancer into responders or non-responders to a treatment with PD-1 and/or PD-L1 inhibitors.

## Description

### FIELD OF THE INVENTION

The present invention refers to the medical field. Particularly, it refers to an *in vitro* method for predicting the response of patients suffering from cancer to a treatment with PD-1 and/or PD-L1 inhibitors. It also refers to an *in vitro* method for recommending whether to treat patients suffering from cancer with PD-1 and/or PD-L1 inhibitors, or for classifying patients suffering from cancer into responders or non-responders to a treatment with PD-1 and/or PD-L1 inhibitors.

### STATE OF THE ART

Immunotherapy is one of the most promising cancer treatments due to its differential mechanism of action, long lasting effects and the fact that it improves overall survival in a percentage of patients. Immune checkpoint inhibitor (ICI) agents constitute the most thoroughly investigated class of immunotherapy. They aim to stimulate the immune response of patients against cancer by targeting programmed cell death protein 1 (PD-1), programmed cell death ligand 1 (PD-L1) or cytotoxic T lymphocyte-associated protein 4 (CTLA-4). The existing PD-1, PD-L1 and CTLA-4 inhibitors are summarised below.

| **PD-1 inhibitors** | **PD-L1 inhibitors** | **CTLA-4 inhibitors** |
|---|---|---|
| Pembrolizumab | Atezolizumab | Ipilimumab |
| Nivolumab | Avelumab | Tremelimumab |
| Cemiplimab | Durvalumab | |
| Dostarlimab | | |

In recent years, immunotherapy has been incorporated in the management of non-small cell lung cancer (NSCLC), which accounts for 80-90% of all lung cancer cases worldwide, leading to an improvement in patients' survival in comparison with chemotherapy. One of the most employed ICIs in advanced NSCLC patients is pembrolizumab, an anti-PD-1 receptor that binds to the PD-1 receptors present in T cells, thereby blocking its binding to the PD-1 ligand (PD-L1) present on the surface tumour cells. Blocking this interaction allows T cells to kill tumour cells. For years, the selection of patients who received immunotherapy was based on PD-L1 expression on tumour tissue. Nevertheless, posterior studies reported that pembrolizumab in combination with chemotherapy led to a significant increase in survival, independently of PD-L1 expression, showing the lack of potential value of PD-L1 expression in tumour tissue to predict the response to PD-1 inhibitors.

However, although ICI therapies have improved patient outcomes in comparison with chemotherapy, clinical use of immunotherapies faces several challenges related to both efficacy and safety. Regarding efficacy, a large group of patients do not respond to ICI treatment (primary resistance); large phase III studies of ICIs combined with chemotherapy reported overall response rates of 47-63% at best. Regarding safety, adverse reactions to ICI treatment, as well as an accelerated progression, denominated hyperprogression, have been reported.

In this context, the management of NSCLC with ICIs requires the identification of new and robust biomarkers to select patients who will benefit from immunotherapy. Liquid biopsy has emerged as an alternative for discovering new biomarkers that could help clinicians to select patients who will benefit from ICIs treatment. Numerous studies focus on potential biomarkers in blood such as circulating tumour cells (CTCs), circulating tumour DNA (ctDNA), circulating free DNA (cfDNA), exosomes, miRNA or proteins among others. Compared with tumour specimens, liquid biopsy samples have the advantage of being easily accessible, avoiding the limitations of the restricted access to tissue and allowing sequential analysis and monitoring during the disease. In addition, liquid biopsy samples are representative of both primary tumour and metastasis. Next-generation sequencing analyses have opened the door to the identification of alterations associated to therapy response and to the design of new possible therapeutic targets. However, given the multifactorial nature of cancer, other approaches - such as proteomic analyses - are also required for a more comprehensive understanding.

In the last decades, proteomic technologies have advanced in terms of instrumentation improvements, sample preparation and computational analyses, allowing us to identify and quantify nearly all expressed proteins in a single experiment. An emerging strategy named SWATH-MS (sequential window acquisition of all theoretical fragment-ion spectra-mass spectrometry) supports quantitative analysis of circulating proteins with high quantitative consistency and accuracy.

So, there is an unmet medical need of finding strategies aimed at identifying patients who will benefit from immunotherapy. In particular, there is a need to identify cancer patients that may benefit from a treatment with PD-1 and/or PD-L1 inhibitors. The present invention is focused on solving this problem and an effective *in vitro* method for predicting the response of patients suffering from cancer to a treatment with PD-1 and/or PD-L1 inhibitors is herein provided. It also provides a method for recommending whether to treat patients suffering from cancer with PD-1 and/or PD-L1 inhibitors, or for classifying patients suffering from cancer into responders or non-responders to a treatment with PD-1 and/or PD-L1 inhibitors.

### DESCRIPTION OF THE INVENTION

### Brief description of the invention

The present invention refers to an *in vitro* method for predicting the response of patients suffering from cancer to a treatment with PD-1 and/or PD-L1 inhibitors, and to an *in vitro* method for recommending whether to treat patients suffering from cancer with PD-1 and/or PD-L1 inhibitors, or for classifying patients suffering from cancer into responders or non-responders to a treatment with PD-1 and/or PD-L1 inhibitors.

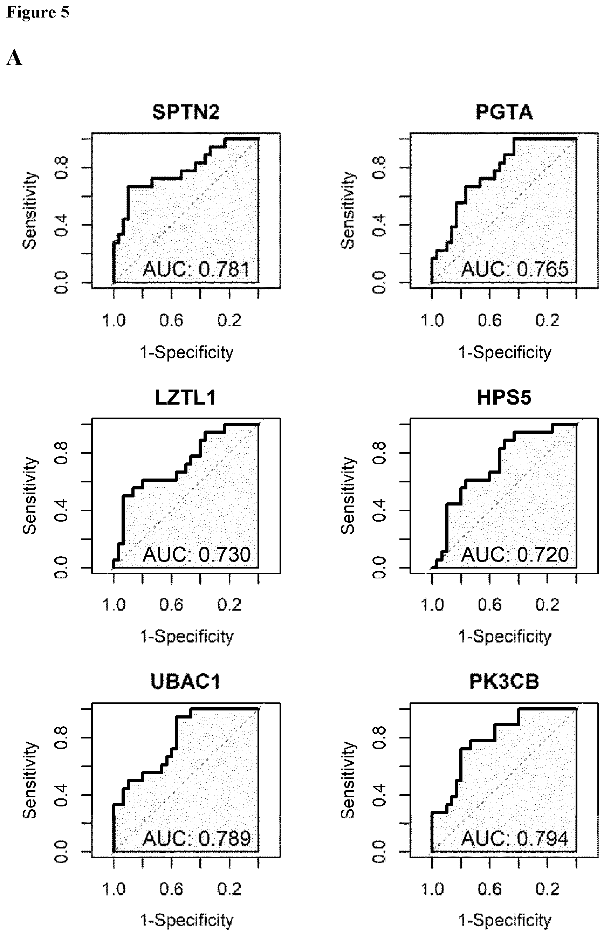

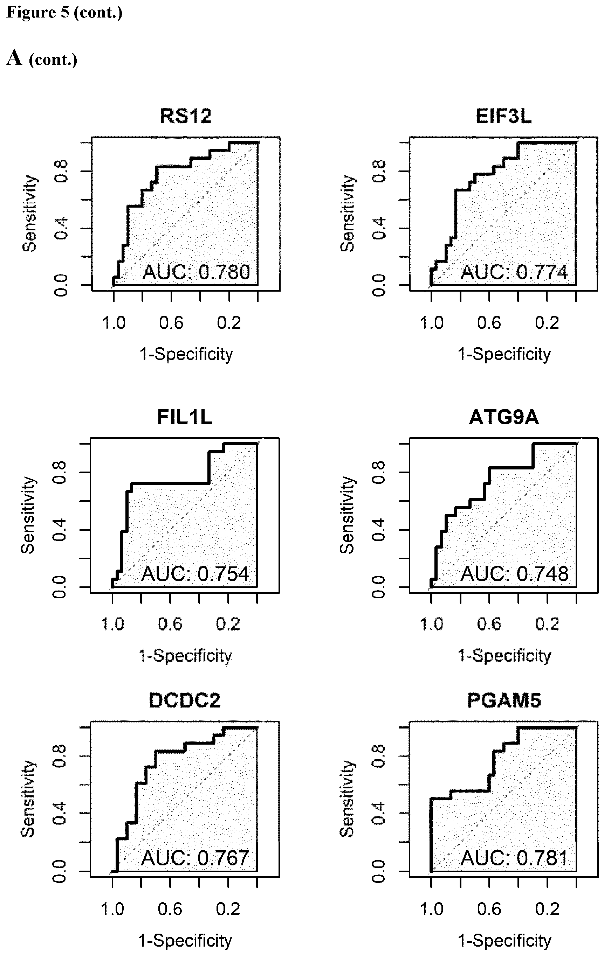

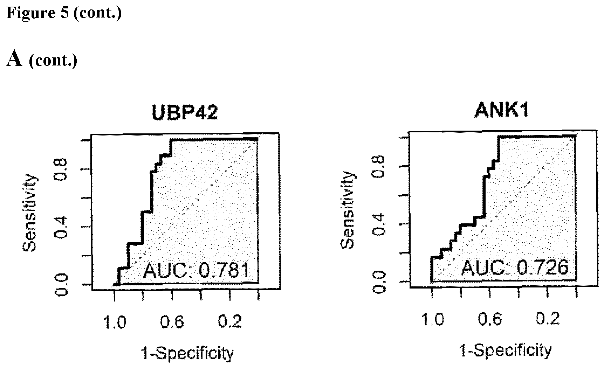

The inventors of the present invention hypothesized that plasma proteins could serve as a prognostic biomarker in NSCLC patients under first-line immunotherapy. To test their hypothesis, they performed a proteomic analysis of plasma samples from patients with advanced NSCLC prior to the start of an anti-PD-1 treatment with pembrolizumab. This led to the identification of fourteen proteins (SPTN2, PGTA, FIL1L, ATG9A, LZTL1, HPS5, DCDC2, PGAM5, UBAC1, PK3CB, UBP42, ANK1, RS12 and EIF3L) that enabled the discrimination between responders and non-responders to pembrolizumab treatment, yielding areas under the ROC curves (AUCs) of 0.72-0.79 (**Figure 5A**).

The Uniprot references of the selected proteins are specified below.

| **Protein** | **Uniprot reference** |
|---|---|
| SPTN2 | O15020 |
| PGTA | Q92696 |
| FELIL | Q4L180 |
| ATG9A | Q7Z3C6 |
| LZTL1 | Q9NQ48 |
| HPS5 | Q9UPZ3 |
| DCDC2 | Q9UHG0 |
| PGAM5 | Q96HS1 |
| UBAC1 | Q9BSL1 |
| PK3CB | P42338 |
| UBP42 | Q9H9J4 |
| ANK1 | P16157 |
| RS 12 | P25398 |
| EIF3L | Q9Y262 |

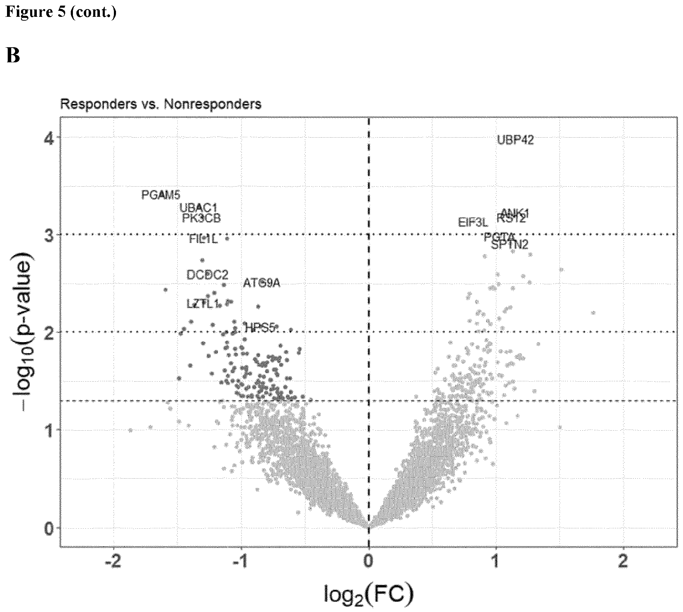

Among them, eight displayed a significantly higher expression in the non-responder group and six in the responder group (**Figure 5B**).

Further analysis revealed that several combinations, comprising from two to fourteen of the selected proteins, displayed unexpectedly higher AUCs than those provided by any of the individual biomarkers alone. Among them, over 6000 combinations were associated to AUCs above 0.8, some of which are represented in **Table 1.** For instance, combination NO: 1 (consisting of proteins SPTN2, PGTA, FIL1L, ATG9A, LZTL1, HPS5 and DCDC2) and combination NO: 2 (consisting of proteins PGAM5, UBAC1, PK3CB, UBP42, ANK1, RS12 and EIF3L) showed an AUC = 1, evidencing their improved efficacy to discriminate between responder and non-responders **(Table 1**). In addition, multiple combinations comprising from two to fourteen of the selected proteins also yielded unexpectedly high AUCs, for instance combination NO: 112 (consisting of proteins FIL1L and ATG9A, AUC = 0.915), combination NO: 126 (consisting of proteins RS12 and PGAM5, AUC = 0.906), or combination NO: 129 (consisting of proteins PGAM5 and EIF3L, AUC = 0.904).

These results can be seen as a proof of concept that makes plausible the use of the selected proteins or combinations thereof as reliable biomarkers for predicting the response of cancer patients to a PD-1 and PDL-1 inhibitor treatment.

**Table 1: Some of the combinations of the selected fourteen proteins.**

| **Combination NO** | **Proteins in the combination** | **AUC** |
|---|---|---|
| 1 | FIL1L+ATG9A+LZTL1+HPS5+DCDC2+SPTN2+PGTA | 1.000 |
| 2 | SPTN2+PGTA+FIL1L+ATG9A+LZTL1+HPS5+DCDC2 | 1.000 |
| 3 | FIL1L+ATG9A+LZTL1+HPS5+DCDC2+PGTA | 0.991 |
| 4 | FIL1L+ATG9A+LZTL1+HPS5+PGTA | 0.987 |
| 5 | FIL1L+ATG9A+LZTL1+HPS5+DCDC2+SPTN2 | 0.987 |
| 6 | FIL1L+ATG9A+LZTL1+HPS5+SPTN2+PGTA | 0.987 |
| 7 | FIL1L+ATG9A+LZTL1+HPS5+DCDC2 | 0.985 |
| 8 | FIL1L+ATG9A+HPS5+SPTN2+PGTA | 0.985 |
| 9 | FIL1L+ATG9A+HPS5+DCDC2+SPTN2+PGTA | 0.985 |
| 10 | FIL1L+ATG9A+HPS5+PGTA | 0.983 |
| 11 | FIL1L+ATG9A+HPS5+DCDC2+PGTA | 0.983 |
| 12 | FIL1L+ATG9A+LZTL1+HPS5 | 0.980 |
| 13 | FIL1L+ATG9A+LZTL1+HPS5+SPTN2 | 0.980 |
| 14 | ANK1+RS12+PGAM5+EIF3L+UBAC1+UBP42 | 0.978 |
| 15 | RS12+PGAM5+EIF3L+UBAC1+PK3CB+UBP42 | 0.978 |
| 16 | ANK1+RS12+PGAM5+EIF3L+UBAC1+PK3CB+UBP42 | 0.978 |
| 17 | FIL1L+ATG9A+HPS5+DCDC2 | 0.976 |
| 18 | ANK1+PGAM5+EIF3L+UBAC1+UBP42 | 0.976 |
| 19 | FIL1L+ATG9A+HPS5 | 0.974 |
| 20 | FIL1L+ATG9A+HPS5+DCDC2+SPTN2 | 0.974 |
| 21 | ANK1+RS12+PGAM5+EIF3L+PK3CB+UBP42 | 0.972 |
| 22 | ANK1+RS12+PGAM5+UBAC1+UBP42 | 0.970 |
| 23 | RS12+PGAM5+EIF3L+PK3CB+UBP42 | 0.970 |
| 24 | ANK1+RS12+PGAM5+UBAC1+PK3CB+UBP42 | 0.970 |
| 25 | ANK1+PGAM5+EIF3L+UBAC1+PK3CB+UBP42 | 0.970 |
| 26 | FIL1L+ATG9A+HPS5+SPTN2 | 0.970 |
| 27 | ANK1+RS12+PGAM5+EIF3L+UBP42 | 0.969 |
| 28 | ANK1+PGAM5+UBAC1+PK3CB+UBP42 | 0.969 |
| 29 | ANK1+RS12+PGAM5+EIF3L+UBAC1+PK3CB | 0.969 |
| 30 | RS12+PGAM5+EIF3L+UBAC1+UBP42 | 0.967 |
| 31 | RS12+PGAM5+EIF3L+UBP42 | 0.963 |
| 32 | ANK1+PGAM5+EIF3L+UBAC1+PK3CB | 0.963 |
| 33 | ANK1+EIF3L+UBAC1+PK3CB+UBP42 | 0.963 |
| 34 | ANK1+PGAM5+UBAC1+UBP42 | 0.961 |
| 35 | ANK1+RS12+PGAM5+EIF3L+UBAC1 | 0.961 |
| 36 | ANK1+EIF3L+UBAC1+PK3CB | 0.959 |
| 37 | RS12+PGAM5+EIF3L+UBAC1+PK3CB | 0.959 |
| 38 | RS12+PGAM5+UBAC1+PK3CB+UBP42 | 0.959 |
| 39 | ANK1+RS12+EIF3L+UBAC1+PK3CB+UBP42 | 0.959 |
| 40 | ANK1+RS12+PGAM5+PK3CB+UBP42 | 0.957 |
| 41 | FIL1L+ATG9A+SPTN2+PGTA | 0.957 |
| 42 | FIL1L+ATG9A+DCDC2+SPTN2+PGTA | 0.956 |
| 43 | ANK1+PGAM5+EIF3L+UBAC1 | 0.956 |
| 44 | ANK1+RS12+EIF3L+UBAC1+UBP42 | 0.956 |
| 45 | FIL1L+ATG9A+PGTA | 0.954 |
| 46 | ANK1+EIF3L+UBAC1+UBP42 | 0.954 |
| 47 | FIL1L+ATG9A+LZTL1+SPTN2+PGTA | 0.952 |
| 48 | FIL1L+ATG9A+LZTL1+DCDC2+SPTN2+PGTA | 0.952 |
| 49 | ANK1+RS12+PGAM5+UBP42 | 0.952 |
| 50 | RS12+PGAM5+EIF3L+PK3CB | 0.952 |
| 51 | PGAM5+EIF3L+UBAC1+PK3CB+UBP42 | 0.952 |
| 52 | RS12+PGAM5+EIF3L | 0.950 |
| 53 | PGAM5+EIF3L+UBAC1+UBP42 | 0.950 |
| 54 | ANK1+RS12+EIF3L+UBAC1+PK3CB | 0.950 |
| 55 | ANK1+PGAM5+EIF3L+PK3CB+UBP42 | 0.950 |
| 56 | ANK1+RS12+PGAM5+EIF3L | 0.948 |
| 57 | RS12+PGAM5+EIF3L+UBAC1 | 0.948 |
| 58 | ANK1+RS12+PGAM5+EIF3L+PK3CB | 0.948 |
| 59 | FIL1L+ATG9A+DCDC2+PGTA | 0.948 |
| 60 | RS12+PGAM5+PK3CB+UBP42 | 0.946 |
| 61 | ANK1+RS12+UBAC1+PK3CB+UBP42 | 0.946 |
| 62 | FIL1L+ATG9A+LZTL1+PGTA | 0.946 |
| 63 | FIL1L+ATG9A+LZTL1+DCDC2+PGTA | 0.946 |
| 64 | RS12+PGAM5+UBAC1+PK3CB | 0.944 |
| 65 | PGAM5+EIF3L+UBAC1+PK3CB | 0.944 |
| 66 | FIL1L+ATG9A+LZTL1+DCDC2+SPTN2 | 0.944 |
| 67 | FIL1L+ATG9A+LZTL1+SPTN2 | 0.943 |
| 68 | ANK1+PGAM5+EIF3L+UBP42 | 0.943 |
| 69 | ANK1+UBAC1+PK3CB+UBP42 | 0.943 |
| 70 | RS12+PGAM5+UBAC1+UBP42 | 0.943 |
| 71 | ANK1+RS12+PGAM5+UBAC1+PK3CB | 0.943 |
| 72 | ANK1+PGAM5+PK3CB+UBP42 | 0.941 |
| 73 | ANK1+RS12+PGAM5+UBAC1 | 0.937 |
| 74 | EIF3L+UBAC1+PK3CB+UBP42 | 0.937 |
| 75 | RS12+EIF3L+UBAC1+PK3CB+UBP42 | 0.937 |
| 76 | ATG9A+LZTL1+HPS5+SPTN2+PGTA | 0.937 |
| 77 | ANK1+UBAC1+UBP42 | 0.935 |
| 78 | ANK1+RS12+UBAC1+UBP42 | 0.935 |
| 79 | ANK1+PGAM5+EIF3L+PK3CB | 0.933 |
| 80 | ANK1+PGAM5+UBAC1+PK3CB | 0.933 |
| 81 | ANK1+RS12+EIF3L+UBAC1 | 0.931 |
| 82 | PGAM5+EIF3L+PK3CB+UBP42 | 0.931 |
| 83 | PGAM5+UBAC1+PK3CB+UBP42 | 0.931 |
| 84 | FIL1L+ATG9A+DCDC2+SPTN2 | 0.931 |
| 85 | ATG9A+LZTL1+HPS5+DCDC2+SPTN2+PGTA | 0.931 |
| 86 | FIL1L+ATG9A+SPTN2 | 0.928 |
| 87 | ANK1+PGAM5+UBP42 | 0.928 |
| 88 | RS12+PGAM5+PK3CB | 0.928 |
| 89 | RS12+PGAM5+UBP42 | 0.928 |
| 90 | PGAM5+EIF3L+UBP42 | 0.928 |
| 91 | ANK1+RS12+PGAM5+PK3CB | 0.928 |
| 92 | RS12+EIF3L+UBAC1+PK3CB | 0.928 |
| 93 | ANK1+RS12+UBAC1+PK3CB | 0.926 |
| 94 | ANK1+EIF3L+UBAC1 | 0.924 |
| 95 | PGAM5+EIF3L+UBAC1 | 0.924 |
| 96 | FIL1L+ATG9A+LZTL1 | 0.924 |
| 97 | FIL1L+ATG9A+LZTL1+DCDC2 | 0.924 |
| 98 | ATG9A+LZTL1+HPS5+PGTA | 0.924 |
| 99 | ATG9A+HPS5+SPTN2+PGTA | 0.924 |
| 100 | PGAM5+EIF3L+PK3CB | 0.922 |
| 101 | RS12+EIF3L+UBAC1+UBP42 | 0.922 |
| 102 | RS12+PGAM5+UBAC1 | 0.920 |
| 103 | EIF3L+UBAC1+PK3CB | 0.920 |
| 104 | ATG9A+LZTL1+HPS5+DCDC2+PGTA | 0.920 |
| 105 | ATG9A+HPS5+DCDC2+SPTN2+PGTA | 0.920 |
| 106 | FIL1L+ATG9A+DCDC2 | 0.919 |
| 107 | ANK1+PGAM5+EIF3L | 0.919 |
| 108 | EIF3L+UBAC1+UBP42 | 0.919 |
| 109 | ANK1+PGAM5+UBAC1 | 0.917 |
| 110 | ANK1+UBAC1+PK3CB | 0.917 |
| 111 | RS12+UBAC1+PK3CB+UBP42 | 0.917 |
| 112 | FIL1L+ATG9A | 0.915 |
| 113 | ATG9A+DCDC2+SPTN2+PGTA | 0.915 |
| 114 | FIL1L+LZTL1+HPS5+SPTN2+PGTA | 0.915 |
| 115 | FIL1L+LZTL1+HPS5+DCDC2+SPTN2+PGTA | 0.915 |
| 116 | ANK1+RS12+PGAM5 | 0.915 |
| 117 | ATG9A+LZTL1+DCDC2+SPTN2+PGTA | 0.913 |
| 118 | RS12+EIF3L+UBAC1 | 0.913 |
| 119 | PGAM5+UBAC1+UBP42 | 0.913 |
| 120 | ANK1+RS12+EIF3L+PK3CB+UBP42 | 0.911 |
| 121 | ATG9A+LZTL1+SPTN2+PGTA | 0.911 |
| 122 | FIL1L+HPS5+DCDC2+SPTN2+PGTA | 0.909 |
| 123 | ANK1+PGAM5+PK3CB | 0.907 |
| 124 | ATG9A+HPS5+DCDC2+PGTA | 0.906 |
| 125 | FIL1L+LZTL1+DCDC2+SPTN2+PGTA | 0.906 |
| 126 | RS12+PGAM5 | 0.906 |
| 127 | FIL1L+DCDC2+SPTN2+PGTA | 0.904 |
| 128 | ATG9A+LZTL1+HPS5+DCDC2+SPTN2 | 0.904 |
| 129 | PGAM5+EIF3L | 0.904 |
| 130 | FIL1L+HPS5+SPTN2+PGTA | 0.900 |
| 131 | FIL1L+HPS5+DCDC2+SPTN2 | 0.898 |
| 132 | ATG9A+LZTL1+PGTA | 0.896 |
| 133 | ATG9A+LZTL1+HPS5+SPTN2 | 0.896 |
| 134 | ATG9A+LZTL1+DCDC2+PGTA | 0.896 |
| 135 | ATG9A+HPS5+DCDC2+SPTN2 | 0.896 |
| 136 | LZTL1+HPS5+SPTN2+PGTA | 0.896 |
| 137 | ATG9A+HPS5+SPTN2 | 0.894 |
| 138 | FIL1L+HPS5+DCDC2+PGTA | 0.894 |
| 139 | ATG9A+HPS5+PGTA | 0.893 |
| 140 | FIL1L+LZTL1+HPS5+SPTN2 | 0.893 |
| 141 | FIL1L+LZTL1+HPS5+DCDC2+SPTN2 | 0.893 |
| 142 | ATG9A+SPTN2+PGTA | 0.891 |
| 143 | LZTL1+SPTN2+PGTA | 0.891 |
| 144 | FIL1L+LZTL1+HPS5+DCDC2+PGTA | 0.891 |
| 145 | LZTL1+HPS5+DCDC2+SPTN2+PGTA | 0.891 |
| 146 | FIL1L+LZTL1+HPS5+PGTA | 0.889 |
| 147 | FIL1L+LZTL1+SPTN2+PGTA | 0.889 |
| 148 | HPS5+DCDC2+SPTN2+PGTA | 0.889 |
| 149 | ATG9A+DCDC2+PGTA | 0.887 |
| 150 | ATG9A+LZTL1+DCDC2+SPTN2 | 0.885 |
| 151 | FIL1L+LZTL1+SPTN2 | 0.880 |
| 152 | ATG9A+LZTL1+SPTN2 | 0.878 |
| 153 | FIL1L+HPS5+SPTN2 | 0.876 |
| 154 | FIL1L+LZTL1+DCDC2+SPTN2 | 0.876 |
| 155 | FIL1L+SPTN2+PGTA | 0.874 |
| 156 | ATG9A+LZTL1+HPS5 | 0.874 |
| 157 | HPS5+SPTN2+PGTA | 0.874 |
| 158 | DCDC2+SPTN2+PGTA | 0.874 |
| 159 | ATG9A+LZTL1+HPS5+DCDC2 | 0.872 |
| 160 | LZTL1+DCDC2+SPTN2+PGTA | 0.872 |
| 161 | FIL1L+LZTL1+HPS5 | 0.870 |
| 162 | LZTL1+HPS5+SPTN2 | 0.869 |
| 163 | FIL1L+LZTL1+HPS5+DCDC2 | 0.869 |
| 164 | FIL1L+DCDC2+SPTN2 | 0.867 |
| 165 | ATG9A+DCDC2+SPTN2 | 0.865 |
| 166 | FIL1L+SPTN2 | 0.863 |
| 167 | LZTL1+HPS5+DCDC2+SPTN2 | 0.863 |
| 168 | FIL1L+HPS5+DCDC2 | 0.861 |
| 169 | FIL1L+LZTL1+DCDC2+PGTA | 0.861 |
| 170 | HPS5+DCDC2+SPTN2 | 0.859 |
| 171 | FIL1L+HPS5+PGTA | 0.857 |
| 172 | ATG9A+HPS5+DCDC2 | 0.854 |
| 173 | ATG9A+HPS5 | 0.852 |
| 174 | FIL1L+LZTL1+PGTA | 0.852 |
| 175 | LZTL1+HPS5+DCDC2+PGTA | 0.848 |
| 176 | FIL1L+DCDC2+PGTA | 0.846 |
| 177 | LZTL1+DCDC2+SPTN2 | 0.846 |
| 178 | FIL1L+HPS5 | 0.844 |
| 179 | DCDC2+SPTN2 | 0.844 |
| 180 | ATG9A+SPTN2 | 0.843 |
| 181 | SPTN2+PGTA | 0.841 |
| 182 | LZTL1+SPTN2 | 0.839 |
| 183 | HPS5+DCDC2+PGTA | 0.839 |
| 184 | ATG9A+PGTA | 0.835 |
| 185 | FIL1L+LZTL1+DCDC2 | 0.831 |
| 186 | LZTL1+HPS5+PGTA | 0.831 |
| 187 | FIL1L+DCDC2 | 0.828 |
| 188 | LZTL1+DCDC2+PGTA | 0.824 |
| 189 | DCDC2+PGTA | 0.822 |
| 190 | FIL1L+PGTA | 0.819 |
| 191 | HPS5+SPTN2 | 0.819 |
| 192 | FIL1L+LZTL1 | 0.817 |
| 193 | LZTL1+HPS5 | 0.817 |
| 194 | HPS5+DCDC2 | 0.817 |
| 195 | ATG9A+LZTL1 | 0.815 |
| 196 | HPS5+PGTA | 0.813 |
| 197 | LZTL1+PGTA | 0.809 |
| 198 | ATG9A+LZTL1+DCDC2 | 0.807 |
| 199 | LZTL1+HPS5+DCDC2 | 0.807 |

So, the **first** embodiment of the invention refers to an *in vitro* method for predicting the response of patients suffering from cancer to a treatment with PD-1 and/or PD-L1 inhibitors, which comprises a) assessing the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, in a biological sample obtained from a subject, b) wherein a higher concentration level of at least one protein selected from the group consisting of: SPTN2, PGTA, UBP42, ANK 1, RS12 and/or EIF3L, or a higher expression level of the genes encoding those proteins, or a lower concentration level of at least one protein selected from the group consisting of: FIL1L, ATG9A, LZTL1, HPS5, DCDC2, PGAM5, UBAC1 and/or PK3CB, or lower expression level of the genes encoding those proteins, as compared to a pre-established threshold value, is an indication that the subject will respond to the treatment.

In a preferred embodiment, the *in vitro* method comprises a) assessing the concentration level of a combination from two to fourteen proteins selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the subject may be classified as a responder to the treatment with PD-1 and/or PD-L1 inhibitors.

In a preferred embodiment, the *in vitro* method comprises a) assessing the concentration level of at least one of the combinations of proteins of **Table 1,** or the expression level of the genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

In a preferred embodiment, the *in vitro* method comprises a) assessing the concentration level of the combination of the following proteins: SPTN2, PGTA, FIL1L, ATG9A, LZTL1, HPS5 and DCDC2, or the expression level of the genes encoding those proteins, or the combination of the following proteins: PGAM5, UBAC1, PK3CB, UBP42, ANK1, RS12 and EIF3L, or the expression level of the genes encoding those proteins, in a biological sample obtained from a subject, b) processing the concentration or expression levels in order to obtain a score value, and c) wherein a higher score value is obtained, as compared to a pre-established threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

In a preferred embodiment, the biological sample obtained from a subject is blood, serum or plasma.

In a preferred embodiment, the cancer is a solid cancer.

In a preferred embodiment, the cancer is lung cancer, preferably non-small cell lung cancer (NSCLC).

In a preferred embodiment, the PD-1 and/or PD-L1 inhibitor is an antibody, preferably an antibody selected from the list comprising: pembrolizumab, nivolumab, atezolizumab, avelumab, durvalumab, cemiplimab or dostarlimab.

The **second** embodiment of the invention refers to an *in vitro* method for recommending whether to treat patients suffering from cancer with PD-1 and/or PD-L1 inhibitors, or for classifying patients suffering from cancer into responders or non-responders to a treatment with PD-1 and/or PD-L1 inhibitors, which comprises a) assessing the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, in a biological sample obtained from a subject, b) wherein a higher concentration level of at least one protein selected from the group consisting of: SPTN2, PGTA, UBP42, ANK1, RS12 and/or EIF3L, or a higher expression level of the genes encoding those proteins, or a lower concentration level of at least one protein selected from the group consisting of: FIL1L, ATG9A, LZTL1, HPS5, DCDC2, PGAM5, UBAC1 and/or PK3CB, or a lower expression level of the genes encoding those proteins, as compared to a pre-established threshold value, is an indication that the treatment with PD-1 and/or PD-L1 inhibitors is recommended or that the subject may be classified as a responder to the treatment with PD-1 and/or PD-L1 inhibitors.

In a preferred embodiment, the *in vitro* method comprises a) assessing the concentration level of a combination from two to fourteen proteins selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the subject may be classified as a responder to the treatment with PD-1 and/or PD-L1 inhibitors.

In a preferred embodiment, the *in vitro* method comprises a) assessing the concentration level of at least one of the combinations of proteins of **Table 1,** or the expression level of the genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

In a preferred embodiment, the *in vitro* method comprises a) assessing the concentration level of the combination of the following proteins: SPTN2, PGTA, FIL1L, ATG9A, LZTL1, HPS5 and DCDC2, or the expression level of the genes encoding those proteins, or the combination of the following proteins: PGAM5, UBAC1, PK3CB, UBP42, ANK1, RS12 and EIF3L, or the expression level of the genes encoding those proteins, in a biological sample obtained from a subject, b) processing the concentration or expression levels in order to obtain a score value, and c) wherein a higher score value is obtained, as compared to a pre-established threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

In a preferred embodiment, the biological sample obtained from a subject is blood, serum or plasma.

In a preferred embodiment, the cancer is a solid cancer.

In a preferred embodiment, the cancer is lung cancer, preferably non-small cell lung cancer (NSCLC).

In a preferred embodiment, the PD-1 and/or PD-L1 inhibitor is an antibody, preferably an antibody selected from the list comprising: pembrolizumab, nivolumab, atezolizumab, avelumab, durvalumab, cemiplimab or dostarlimab.

PD-1 and/or PD-L1 inhibitors are used in the treatment of a wide variety of cancers. Those treated with pembrolizumab are summarised in **Table 2**. Therefore, the methods described in the first and second embodiments could be applied to a wide variety of cancers, especially those that are generally treated with PD-1 and/or PDL-1 inhibitors.

**Table 2. Tumors treated with pembrolizumab.**

| | |
|---|---|
| **NSCLC** | Stage IB, II, IIIA: Adjuvant treatment + surgery. |
| | Stage III unresectable |
| | Advanced |
| **Melanoma** | Adjuvant therapy |
| | Unresectable |
| | Metastatic |
| **Head and Neck Squamous (HNSCC)** | Unresectable, Recurrent |
| | Metastatic |
| **Urothelial Carcinoma** | Locally advanced |
| | Metastatic |
| **Bladder cancer** | High-Risk Non-muscle Invasive Bladder Cancer (NMIBC) |
| | |
| **Breast cancer** | Advanced TNBC or High-Risk Early-Stage Triple-Negative Breast Cancer (TNBC) |
| **Endometrial cancer** | Advanced MSI-H/dMMR Endometrial Carcinoma |
| **CRC** | Unresectable MSI-H or dMMR colorectal cancer (CRC) |
| | Metastatic MSI-H or dMMR |
| **Renal Cell Carcinoma** | Adjuvant Treatment |
| | Advanced |
| **Other in advanced stages** | Advanced Esophageal or GEJ Carcinoma |
| | Advanced Gastric or GEJ Cancer |
| | Advanced Cervical Cancer |
| | Advanced Merkel Cell Carcinoma (MCC) |
| | Advanced TMB-H Cancers |
| | Advanced Cutaneous Squamous Cell Carcinoma (cSCC) |
| **Others** | Relapsed or Refractory Classical Hodgkin Lymphoma (cHL) Refractory or Relapsed Primary Mediastinal Large B-cell |
| | Lymphoma (PMBCL) |

The PD-1 inhibitors that are approved for the treatment of NSCLS are pembrolizumab, nivolumab and cemiplimab. Nivolumab is often used a second line treatment following chemotherapy, while cemiplimab is often used in combination with chemotherapy.

Small cell lung cancer (SCLC), which is another type of lung cancer, is often treated with nivolumab (PD-1 inhibitor), atezolizumab (PD-L1 inhibitor) and/or durvalumab (PD-L1 inhibitor) in combination with chemotherapy.

A summary of PD-1 inhibitors used in the treatment of other cancers are summarised in **Table 3.**

**Table 3. Summary of PD-1 inhibitors used in cancers other than lung cancer.**

| **PD-1 inhibitors** | **Indication based on tumor type** | |
|---|---|---|
| Pembrolizumab | Summarised in **Table 2**. | |
| Nivolumab | Melanoma | Unresectable or Metastatic |
| | | Adjuvant Treatment |
| | Renal Carcinoma | Advanced |
| | Head and Neck | Recurrent or metastatic |
| | Urothelial | Locally advanced or metastatic urothelial |
| | Colorectal | Metastatic with Microsatellite |
| | | Instability-High (MSI-H) or Mismatch |
| | | Repair Deficient (dMMR) |
| | Hepatocellular | Patients who have been previously treated with sorafenib. |
| Cemiplimab | Cutaneous squamous cell carcinoma | Unresectable or advanced. |
| | Skin cancer called basal cell carcinoma (BCC): | Locally advanced BCC or metastatic. |
| Dostarlimab | Endometrial cancer | Adult patients with mismatch repair deficient (dMMR) recurrent or advanced: |
| | Solid tumors | |

The **third** embodiment of the invention refers to the *in vitro* use of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the genes encoding those proteins, or of a kit consisting of reagents for measuring the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, for predicting the response of patients suffering from cancer to a treatment with PD-1 and/or PD-L1 inhibitors, for recommending whether to treat patients suffering from cancer with PD-1 and/or PD-L1 inhibitors, or for classifying patients suffering from cancer into responders or non-responders to a treatment with PD-1 and/or PD-L1 inhibitors.

A preferred embodiment involves the *in vitro* use a combination from two to fourteen proteins selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or of at least one of the combinations of proteins of **Table 1,** or the combination of the following proteins: SPTN2, PGTA, FIL1L, ATG9A, LZTL1, HPS5 and DCDC2; or PGAM5, UBAC1, PK3CB, UBP42, ANK1, RS12 and EIF3L, or of the expression level of genes encoding those proteins, for predicting the response of patients suffering from cancer to a treatment with PD-1 and/or PD-L1 inhibitors, for recommending whether to treat patients suffering from cancer with PD-1 and/or PD-L1 inhibitors, or for classifying patients suffering from cancer into responders or non-responders to a treatment with PD-1 and/or PD-L1 inhibitors.

In a preferred embodiment, the biological sample obtained from a subject is blood, serum or plasma.

In a preferred embodiment, the cancer is lung cancer, preferably non-small cell lung cancer (NSCLC).

In a preferred embodiment, the PD-1 and/or PD-L1 inhibitor is an antibody, preferably an antibody specifically binding PD-1 and/or PD-L1 selected from the list comprising: pembrolizumab, nivolumab, atezolizumab, avelumab, durvalumab, cemiplimab or dostarlimab.

The **fourth** embodiment of the invention refers to a PD-1 and/or PD-L1 inhibitor, or any pharmaceutical composition comprising thereof, optionally including pharmaceutically acceptable excipients or carriers, and optionally in combination with chemotherapy, for use in the treatment of patients suffering from cancer, wherein the method comprises assessing whether the patient is a responder to the treatment by following any of the methods described in the present invention.

In a preferred embodiment, the cancer is a solid cancer.

In a preferred embodiment, the cancer is lung cancer, preferably non-small cell lung cancer (NSCLC).

In a preferred embodiment, the PD-1 and/or PD-L1 inhibitor is an antibody specifically binding PD-1 and/or PD-L1, preferably an antibody selected from the list comprising: pembrolizumab, nivolumab, atezolizumab, avelumab, durvalumab, cemiplimab or dostarlimab.

The **present** invention also refers to a method for treating patients suffering from cancer, for instance patients suffering from NSCLC, with PD-1 and/or PD-L1 inhibitors, or any pharmaceutical composition comprising thereof, optionally including pharmaceutically acceptable excipients or carriers, and optionally in combination with chemotherapy, wherein the method of treatment comprises a first step for assessing the response of patients suffering from cancer to a treatment with PD-1 and/or PD-L1 inhibitors described in the present invention.

In a preferred embodiment, the cancer is a solid cancer.

In a preferred embodiment, the cancer is lung cancer, preferably non-small cell lung cancer (NSCLC).

In a preferred embodiment, the PD-1 and/or PD-L1 inhibitor is an antibody specifically binding PD-1 and/or PD-L1, preferably an antibody selected from the list comprising: pembrolizumab, nivolumab, atezolizumab, avelumab, durvalumab, cemiplimab or dostarlimab.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the pharmaceutical composition of the invention and that causes no significant adverse toxicological effects to the patient.

### Description of the figures

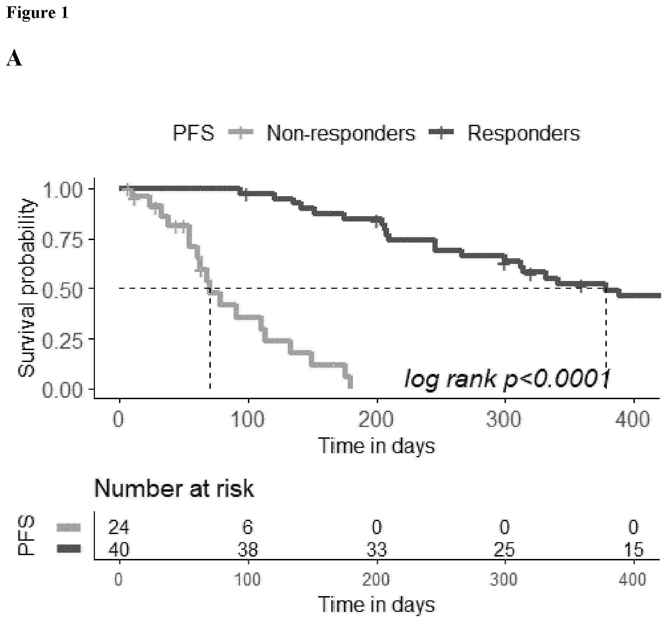

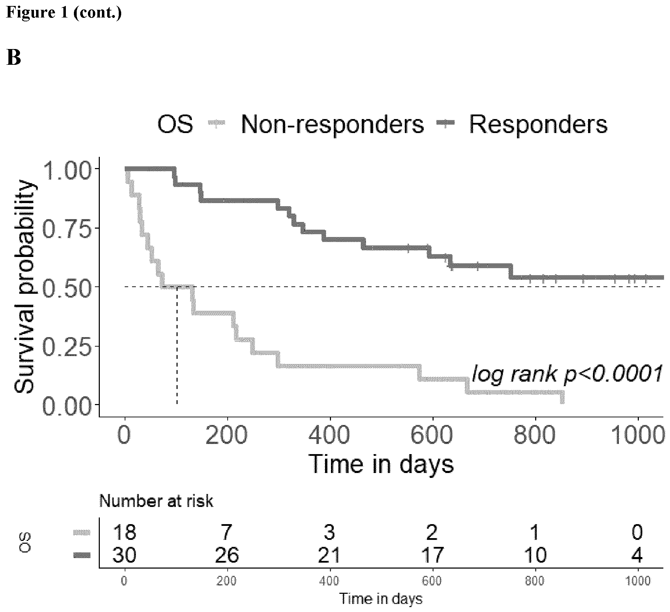

**Figure 1****.** Kaplan-Meier plots show highly significant differences between responders and non-responders to pembrolizumab treatment in **(A)** progression-free survival (PFS) and **(B)** overall survival (OS).

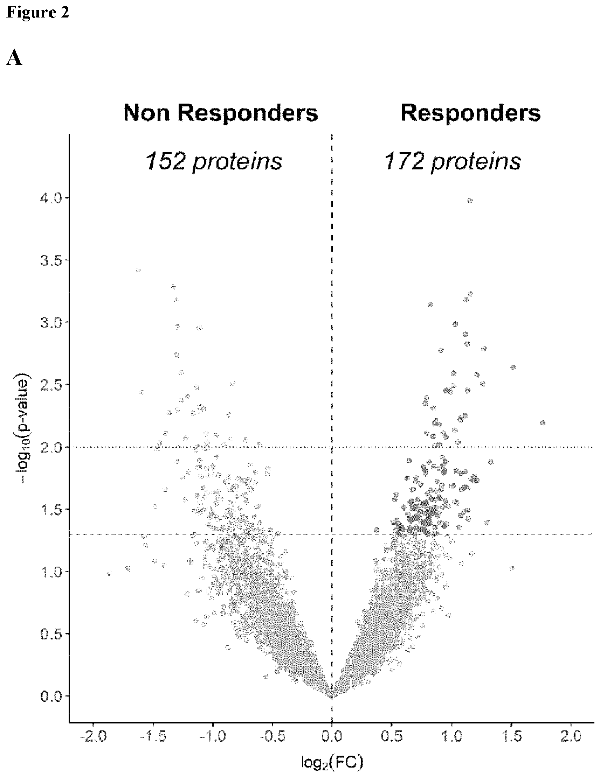

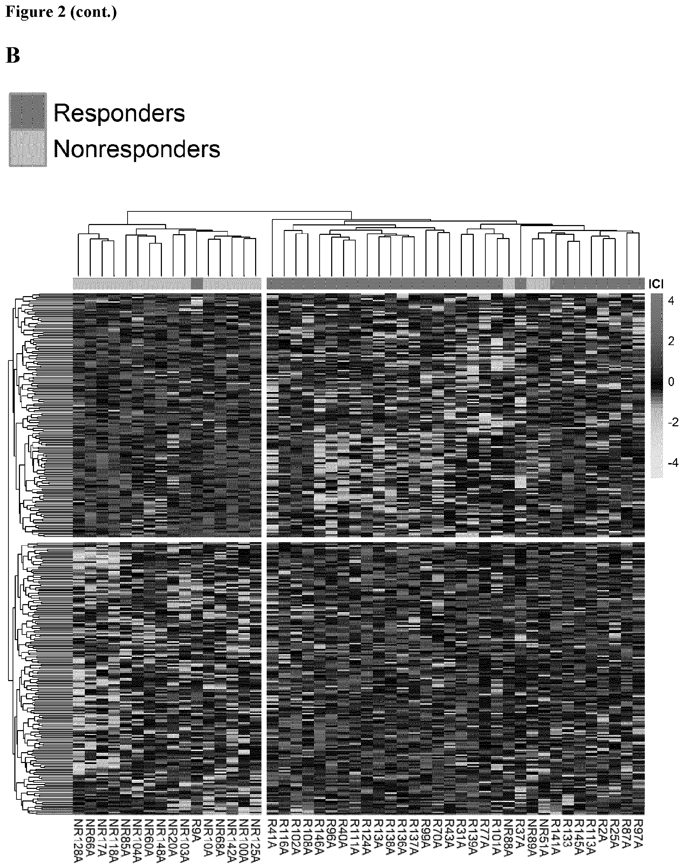

Figure 2. (A) Volcano plot of differentially expressed proteins in plasma from responders versus non-responders at baseline. Proteins that were upregulated or downregulated in the responder group compared to non-responders are shown in blue and yellow, respectively. (B) Expression heatmap of the 324 differentially expressed proteins at baseline (p-value <0.05) showing that the expression pattern of those proteins discriminates between responder and non-responder patients to pembrolizumab therapy. ICI refers to the response obtained after treatment (ICI). Non responders in yellow. Responders in blue.

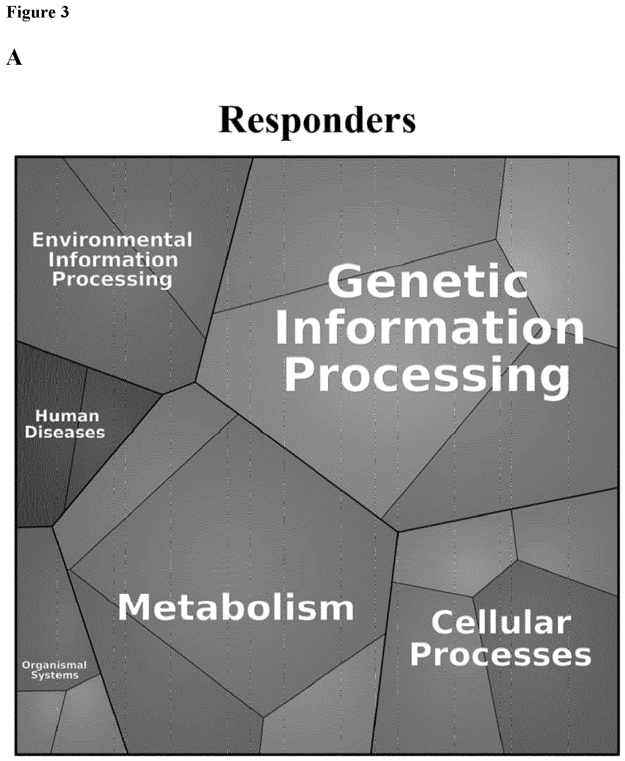

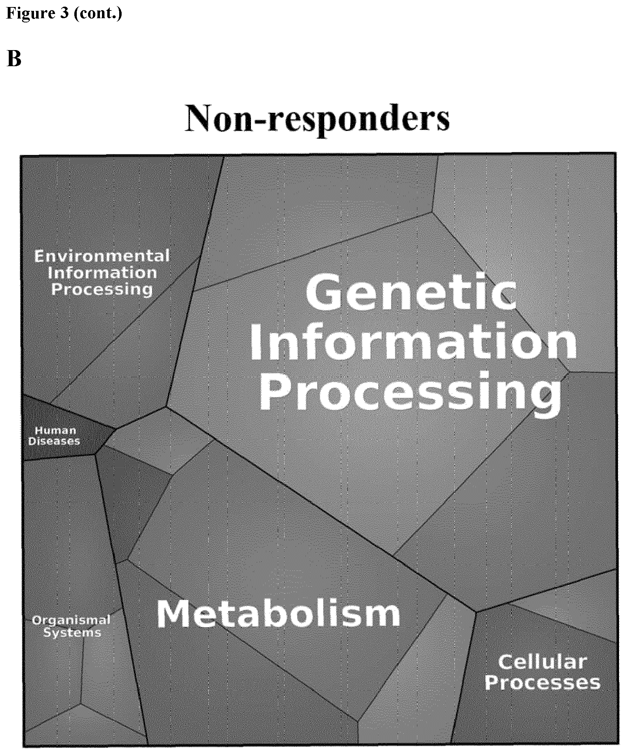

**Figure 3****. Functional enrichment analyses.** Proteomaps showed the functional differences between **(A)** responders and **(B)** non-responders to immunotherapy. Each polygon corresponds to a single KEGG pathway, and its size correlates with the ratio between both groups.

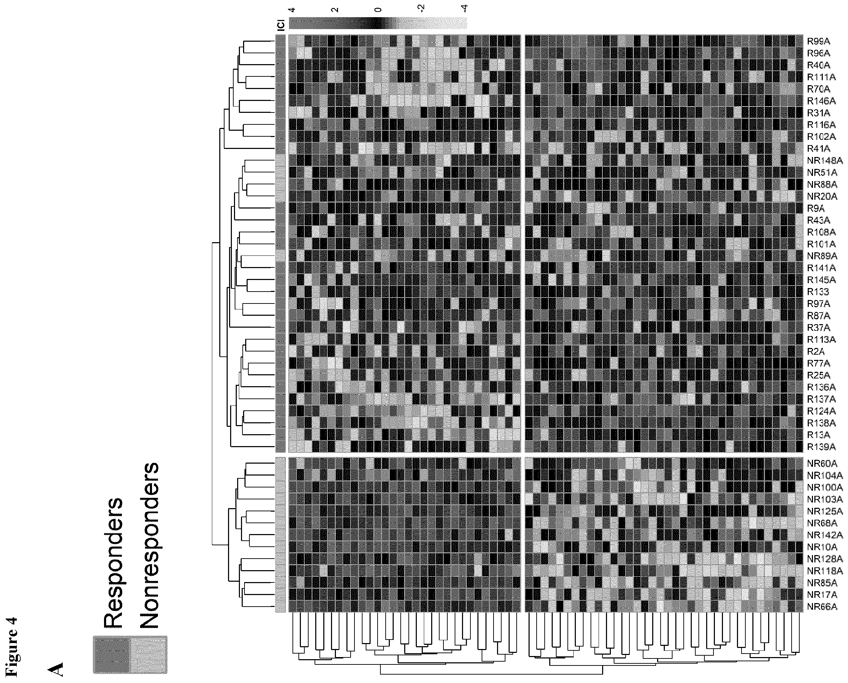

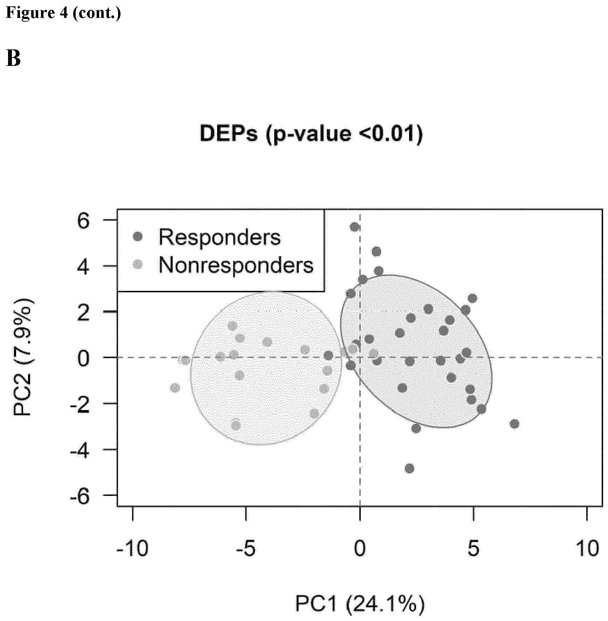

Figure 4. (A) Heatmap of differentially expressed proteins at baseline (p-value <0.01), that discriminate between responder and non-responder patients to Pembrolizumab therapy. **(B)** PCA analysis showing the separation of samples from responders (yellow) and non-responders (blue) to pembrolizumab therapy according the 39 DEPs found by MS/MS analysis.
Figure 5. (A) Receiver operating characteristics (ROCs) curves of selected proteins. **(B)** Volcano plot of differentially expressed proteins at baseline showing our fourteen selected proteins. Proteins that were upregulated or downregulated in the responder group compared to non-responders are shown in yellow and blue, respectively.

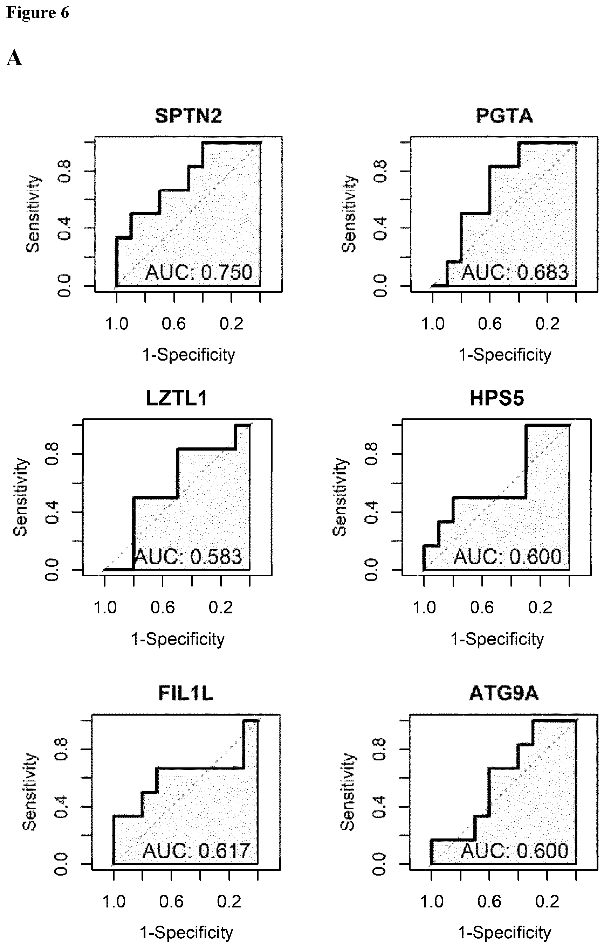

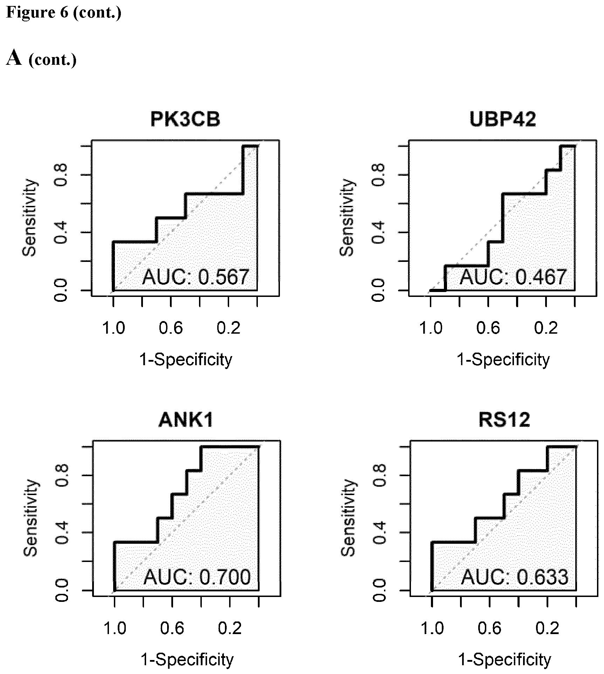

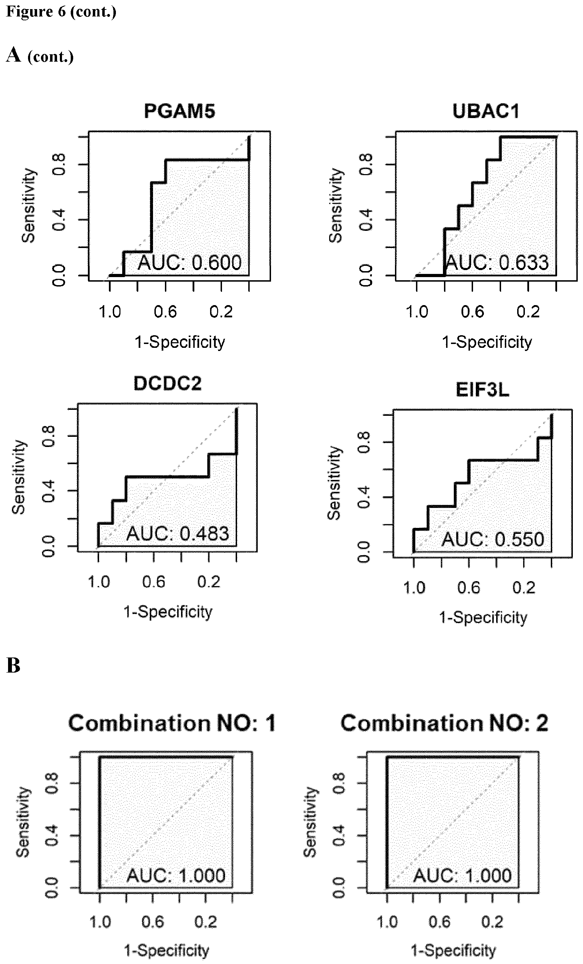

**Figure 6****. Proteins model in the validation cohort. (A)** Receiver operating characteristics (ROCs) curves of the fourteen selected proteins, and of combination NO: 1 and combination NO:2 in the validation cohort. **(B)** ROC analyses of combination NO: 1 and combination NO: 2 presented excellent discrimination rates in the validation (AUC=1 in both cases).

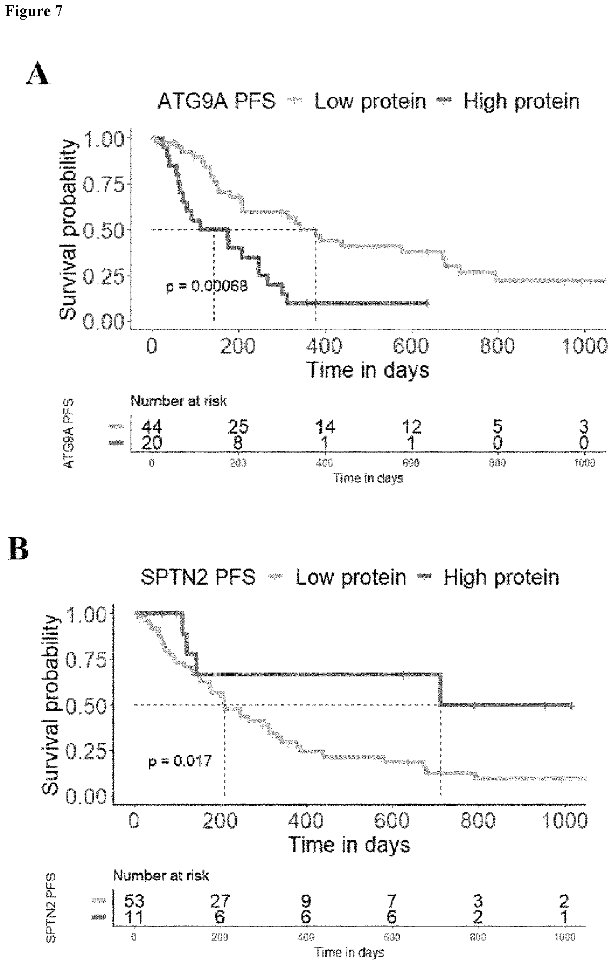

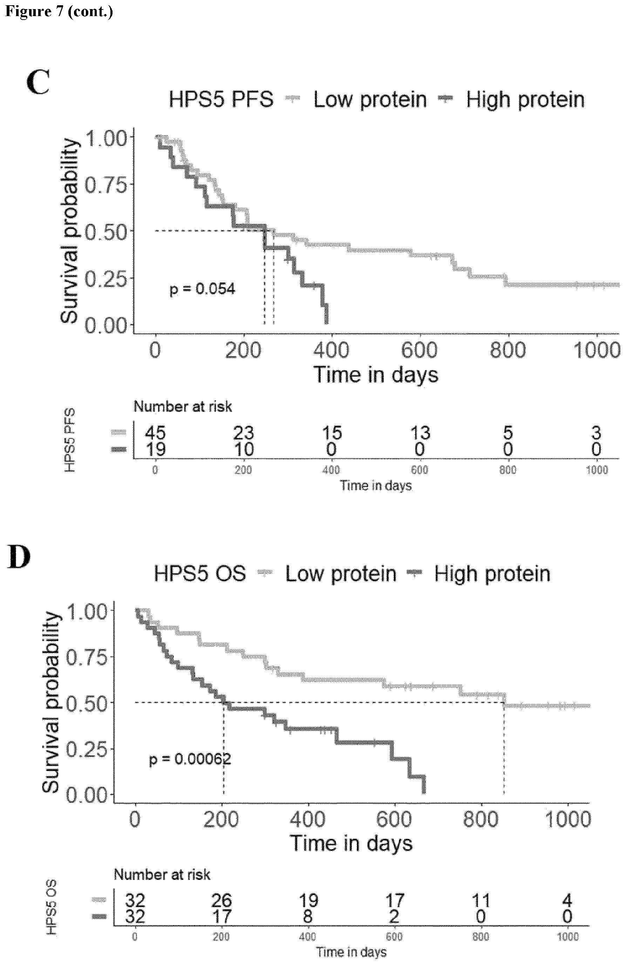

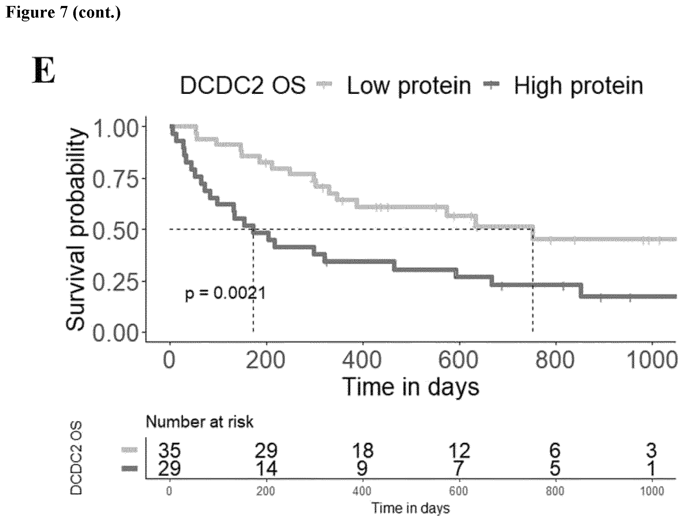

**Figure 7****. Validation of the clinical significance of some of the 14 selected proteins.** Kaplan-Meier survival analysis of ATG9A, SPTN2, HPS5 and DCDC2 proteins levels at baseline. Kaplan-Meier plots of **(A-C)** progression-free survival **(D-E)** and overall survival showed significant associations between proteins levels before start therapy and prognosis in metastatic NSCLC under immunotherapy regimens. Abbreviations: OS, overall survival; PFS, progression free survival.

### Detailed description of the invention

The present invention is illustrated by means of the Examples set below without the intention of limiting its scope of protection.

### Example 1. Material and Methods

### Example 1.1. Patients and blood sample collection

In total, 64 newly diagnosed advanced NSCLC patients treated with pembrolizumab therapy at the Department of Medical Oncology of Complexo Hospitalario Universitario de Santiago de Compostela, were included in the present study. A first cohort (Discovery cohort) included a total of 48 patients with newly diagnosed advanced NSCLC. A validation cohort with 16 new diagnosed advanced NSCLC patients was later on included. The study was performed in accordance with the Declaration of Helsinki (as revised in 2013) and all individuals signed informed consent forms approved by Santiago de Compostela and Lugo Ethics Committee (Ref: 2017/538) prior to enrolling in the study and they could withdraw their consent at any time. Blood samples of 64 patients were collected before the therapy onset. In addition, 171 longitudinal blood samples were collected at 6 weeks and 12 weeks after starting the therapy and at time of progression disease (134 blood samples of the discovery cohort and 37 blood samples of the validation cohort).

Best response to pembrolizumab treatment was based on RECIST1.1, according to the following criteria: Complete response (CR), partial response (PR), stable disease (SD) or progressive disease (PD). Responders were defined as the proportion of patients with CR, PR or SD.

### Example 1.2. Blood sample processing

Ten mL of peripheral blood was obtained by direct venipuncture in Cell Save tubes (Menarini, Silicon Biosystems, Bologna, Italy) and processed within 96 hours after blood collection for the discovery cohort. In the case of the validation cohort, peripheral blood was obtained in EDTA tubes and processed within 6 hours. In both cases, plasma and cellular components were separated by centrifugation at 1,600 g for 10 minutes at room temperature. A second centrifugation was performed at 5,500 g for 10 minutes at room temperature to remove any remaining cellular debris. Plasma samples were aliquoted for storage at -80 °C until use.

### Example 1.3. Plasma preparation for mass spectrometry (MS) analysis

### Example 1.3.1. Plasma abundant proteins depletion

Plasma samples were fractionated prior proteolytic digestion to enrich the spectral library employed in SWATH-MS quantification. For this purpose, aliquots of each sample (30 µl) were depleted with dithiothreitol (DTT). Fresh DTT (500 mM) was mixed with 30 µl of human plasma samples and vortex briefly. Then, samples were incubated until a viscous white precipitate that persisted for 60 min was observed and they were centrifugated at 18,840 g for 20 minutes. Supernatants were transferred to a clean tube.

### Example 1.3.2. Isolation and proteins digestion

In order to make global protein identification, an equal amount of protein from all samples was loaded on a 10% SDS-PAGE gel. The run was stopped as soon as the front had penetrated 3 mm into the resolving gel. The protein band was detected by Sypro-Ruby fluorescent staining (Lonza, Switzerland), excised, and processed for in-gel manual tryptic digestion. Proteins were reduced with DTT 10mM in Ambic 40mM and alkylated with 55 mM iodoacetamide in 50 mM ammonium bicarbonate. Then, the gel pieces were rinsed with 50 mM ammonium bicarbonate in 50% methanol dehydrated by addition of acetonitrile and dried in a SpeedVac. Modified porcine was added to the dry gel pieces at a final concentration of 20 ng/µl in 20mM ammonium bicarbonate, incubating them at 37 °C for 16 h. Peptides were extracted by carrying out three 20 minutes incubations in 40 µL of 60% acetonitrile dissolved in 0.5% HCOOH. The resulting peptide extracts were pooled, concentrated in a SpeedVac, and stored at -20 °C.

### Example 1.4. Protein quantification by SWATH-MS (Sequential Window Acquisition of all Theoretical Mass Spectra)

### Example 1.4.1. Creation of the spectral library - Data-dependent acquisition (DDA)

In order to construct the MS/MS spectral libraries, the peptide solutions from the discovery cohort were analysed by a shotgun data-dependent acquisition (DDA) approach by micro-liquid chromatography-MS/MS. To get a good representation of the peptides and proteins present in all samples, pooled vials of samples from each group (basal n=48, 6 weeks n=35, 12 weeks n=30 and progression disease, n=21) were prepared using equal mixtures of the original samples for the spectral library building. Four µL (4µg) of the pool was separated using Reverse Phase Chromatography. Gradient was created using a micro liquid chromatography system (micro-LC) Ekspert nLC425 (Eksigent Technologies nanoLC 400, Sciex, CA, USA) coupled to high-speed Triple TOF 6600 mass spectrometer (Sciex, CA, USA) with a micro flow source. The chosen analytical column was a silica-based reversed phase column Chrom XP C18 150 × 0.30 mm, 3 mm particle size and 120 Å pore size (Eksigent, Sciex, CA, USA). The trap column was a YMC-TRIART C18 (YMC Technologies, Teknokroma) with a 3 mm particle size and 120 Å pore size, switched on-line with the analytical column. The loading pump delivered a solution of 0.1% formic acid in water at 10 µl/min. The micro-pump generated a flow-rate of 5 µl/min and was operated under gradient elution conditions. Water and acetonitrile, both containing 0.1% formic acid, were used as solvents A and B, respectively. The gradient run consisted of 5% to 95% B for 30 min, 5 min at 90% B and finally 5 min at 5% B for column equilibration, for a total run time of 40 min.

When the peptides eluted, they were directly ionized and injected into a hybrid quadrupole-TOF mass spectrometer Triple TOF 6600 (Sciex, CA, USA) operated with a Data dependent acquisition (DDA) system in positive ion mode. A Micro source (Sciex, CA, USA) was used for the interface between micro-LC and MS, with an application of 2600 V voltage. The acquisition mode consisted of a 250 ms survey MS scan from 400 to 1250 m/z followed by an MS/MS scan from 100 to 1500 m/z (25 ms acquisition time) of the top 65 precursor ions from the survey scan, for a total cycle time of 2.8 seconds. The fragmented precursors were then added to a dynamic exclusion list for 15 seconds; any singly charged ions were excluded from the MS/MS analysis. The instrument was automatically calibrated every 4 hours using external calibrant tryptic peptides from PepCalMix solution (AB SCIEX, Framingham, MA, United States).

The peptide and protein identifications were performed using Protein Pilot software (version 5.0.2, Sciex, CA, USA) searched against a Human specific Uniprot database (https://www.uniprot.org/), specifying iodoacetamide as Cys alkylation and Trypsin as enzyme used in the digestion. The software uses the algorithm Paragon^{™} for database search and Progroup^{™} for data grouping. The false discovery rate (FDR) was set to 1% for both peptides and proteins, using a non-lineal fitting method.

The MS/MS spectra of the identified peptides were then used to generate the spectral library for SWATH peak extraction using the plug-in MS/MSALL with SWATH Acquisition MicroApp (version 2.0, Sciex) for PeakView Software (version 2.2, Sciex, CA, USA). Peptides with a confidence score above 99% (as obtained from Protein Pilot database search) were included in the spectral library.

In addition, a spectral online library called, Human Pan-Human library, which contained 12,046 proteins (https://db.systemsbiology.net/sbeams/cgi/PeptideAtlas/GetDIALibs), was also employed to improve and expand the coverage of the identified NSCLC cancer plasma proteome in our library. The sum of the two libraries constitutes our final library, denominated NSClibrary.

### Example 1.4.2. Relative quantification by SWATH acquisition - Data-independent acquisition (DIA)

SWATH-MS acquisition was performed on a TripleTOF^{®} 6600 LC-MS/MS system (AB Sciex, CA, USA). Samples were analysed using a data-independent acquisition (DIA) method (n=171 samples). Each sample (4 µL) was analysed using the LC-MS equipment and LC gradient described above for building the spectral library but instead using the SWATH-MS acquisition method. The method consisted of repeating a cycle that is composed of the acquisition of 100 TOF MS/MS scans (400 to 1500 m/z, high sensitivity mode, 50 ms acquisition time) of overlapping sequential precursor isolation windows of variable width (1 m/z overlap) covering the 400 to 1250 m/z mass range with a previous TOF MS scan (400 to 1500 m/z, 50 ms acquisition time) for each cycle. Total cycle time was 6.3 seconds. For the sample set, the width of the 100 variable windows was optimized according to the ion density found in the DDA runs using a SWATH variable window calculator worksheet from Sciex. SWATH quantification was attempted for all proteins in the ion library that were identified by ProteinPilot with an FDR below 1%. The DIA raw data were firstly converted into mzMI, format filtered by SWATH Acquisition MicroApp (version 2.0) then analyzed by PeakView (version 2.2), against our spectral NSCLibrary. PeakView computed an FDR and a score for each assigned peptide according to the chromatographic and spectra components; only peptides with an FDR below 1% were used for protein quantitation. The retention time adjustment was performed using the own peptides of the proteins present in the library. From the peptides that were selected for each protein, then the RT of all peptides were realigned in each run according to the iRT peptides spiked of the selected peptides in each sample and eluted along the whole-time axis. The extracted ion chromatograms were then generated for each selected fragment ion; the peak areas for the peptides were obtained by summing the peak areas from the corresponding fragment ions. Protein quantitation was calculated by adding the peak areas of the corresponding peptides Up to ten peptides per protein and seven fragments per peptide were selected, based on signal intensity; any shared and modified peptides were excluded from the processing. Five-minute windows and 30 ppm widths were used to extract the ion chromatograms. Then, these integrated peak areas (processed. mrkvw files from PeakView) were directly exported to the MarkerView 1.3.1 software (AB Sciex, CA, USA) for relative quantitative analysis. The export data will generate three files containing quantitative information about individual ions, the summed intensity of different ions for a particular peptide and the summed intensity of different peptides for a particular protein. MarkerView has been used for analysis of SWATH-MS data reported in other proteomics studies because of its data-independent method of quantitation. MarkerView uses processing algorithms that accurately find chromatographic and spectral peaks direct from the raw SWATH data. Data alignment by MarkerView compensates for minor variations in both mass and retention time values, ensuring that identical compounds in different samples are accurately compared to one another.

### Example 1.5. Quality control and statistical analysis

Proteins or samples with over 70% missing values in the sample set were filtered out. Next, missing values of the remaining proteins were imputed using the RandomForest R package. Proteins values were normalization by quantile normalization. Continues data were compared using t test for independent samples and categorical variables were compared using the Fisher's exact test. Swimmer plot was provided to visualize every patient's therapy response and the time of survival from the diagnoses. Kaplan-Meier method was used to plot the survival curves applying the log rank test. Student's t test was applied to identified differentially-expressed proteins (DEPs), with a p-value< 0.05 and p-value< 0.01. To identify the functions and relevant pathways of the DEPs, we performed gene ontology (GO) using GeneCodis 4. In addition, the Kyoto Encyclopedia of Genes and Genomes (KEGG) pathway of each group was generated and visualize with proteomaps, using a web tool based on the t-test difference values without log2 transformation. A final predictive model to separate responder group to non-responder group was developed. Comparisons of Cox proportional hazard regression models were made using the Akaike information criterion (AIC) technique with a smaller AIC value indicating the better model. A stepwise backward elimination procedure was performed to minimize the AIC. Receiver operating characteristics (ROC) curves were computed based on protein levels, representing the area under the curve (AUC) values, and computing the confidence intervals (CI) at 95% confidence levels. ROC curves were also constructed to evaluate the thresholds of baseline cfDNA levels for PFS and OS analyses. The Mann-Whitney-Wilcoxon U-Test was used to compare proteins levels between groups.

All statistical analyses were performed using GraphPad Prism version 8.0, R version 4.1.1 and SPSS software. The following R packages were used: survival, survminer, ggplot2, pROC, gtsummary, swimplot, MASS and stats.

### Example 2. Results

### Example 2.1. Patients' characteristics

In total, 64 newly diagnosed NSCLC patients undergoing first-line pembrolizumab treatment in monotherapy (n=40) or in combination with chemotherapy (n=24) were included in the study (**Table 4**). Patients were enrolled in two different sub-cohorts: a discovery cohort with 48 NSCLC patients and a validation cohort with 16 NSCLC patients. In global terms, the mean age was 64.7 years (range: 45-80), the majority of patients were males (75%), current or former smokers (87.5%) and had tumours with adenocarcinoma histology (81.25%) **(Table 4**). The Eastern Cooperative Oncology Group Performance Status (ECOG PS) of <1 accounted for 17.2% of patients.

First, the discovery cohort with 48 patients with advanced NSCLC was enrolled. Of them, 18 patients presented progression disease, only 1 patient presented complete response, 20 patients presented partial responses and 9 patients, stable disease. Thus, we divided our cohort into two main groups: 62.5% of patients were denominated responders (including stable disease, partial and complete responders; n=30) and 37.5% of patients were denominated non-responders (progressive disease; n=18) **(Table 4**). Analyses between both groups showed that responders and non-responders to pembrolizumab treatment presented differences in the number of metastatic sites. 61.1% of non-responders had more than two metastatic sites versus 16.7% for the responder's group (Fisher test; p-value< 0.01). In addition, responders and non-responders presented highly significant differences in the time of progression-free survival (PFS, 578 days versus 68 days, respectively) and in the time of overall survival (OS, 1070 days versus 102 days, respectively) (**Table 4**). We did not find any association between responder and non-responder patients for the rest of clinical characteristics at baseline.

In the validation cohort, the analysed samples were taken from 16 patients with advanced NSCLC undergoing first-line pembrolizumab treatment in monotherapy (n=8) or in combination with chemotherapy (n=8) (**Table 4**). 62.5% of patients were denominated responders (including stable disease (n=2) and partial response (n=8)) and 37.5% of patients were denominated non-responders (progressive disease; n =6). The validation cohort presented similar characteristics than the discovery cohort, except sex distribution. Similar to the previous cohort, responders and non-responders presented highly significant differences in the time of PFS and in the time of OS.

Kaplan Meier plots representing the differences in survival between responder and non-responders are shown in **Figure 1****.**

**Table 4. Patients' demographics and clinical characteristics at baseline. SD: standard deviation; ECOG PS: Eastern Cooperative Oncology Group Performance Status; NS, no significative; PFS, progression-free survival; OS, overall survival.**

| **Clinical Characteristics** | **Discovery cohort (n=48)** | | | **Validation cohort (n=16)** | | |
|---|---|---|---|---|---|---|
| | **Responders (n=30) N (%)** | **Non-responders (n=18) N (%)** | **p-value** | **Responders (n=10) N (%)** | **Non-responders (n=6) N (%)** | **p-value** |
| **Mean age ± SD, range** | 64.3 ± 8.2, 50-80 | 61.9 ± 9.5, 45-78 | NS | 71.5 ± 5.4, 60-77 | 63.2 ± 7.6, 51-74 | NS |
| **Sex** | | | | | | |
| Female | 6 (20.0) | 5 (27.8) | NS | 1 (9.1) | 4 (66.7) | **0.03** |
| Male | 24 (80.0) | 13 (72.2) | | 9 (90.9) | 2 (33.3) | |
| **Smoking** | | | | | | |
| Smoker t Former smoker | 25 (83.3) | 16 (88.9) | NS | 9(90) | 6(100) | NS |
| Never | 5 (16.7) | 2 (11.1) | | 1 (10) | 0 (0.0) | |
| **ECOG PS** | | | | | | |
| 0 | 8 (26.7) | 1 (5.6) | NS | 1 (10) | 0 (0.0) | NS |
| 1 | 21 (70.0) | 13 (72.2) | | 10 (90) | 4 (66.7) | |
| 2 | 1 (3.3) | 4 (22.2) | | 0 (0.0) | 2 (33.3) | |
| **Histology** | | | | | | |
| Adenocarcinoma | 26 (86.7) | 16 (88.9) | NS | 6 (60) | 4 (66.6) | NS |
| Squamous cell carcinoma | 3 (10.0) | 2 (11.1) | | 4(40) | 1 (16.7) | |
| Others | 1 (3.3) | 0 (0.0) | | a (0.0) | 1 (16.7) | |
| **Number of metastatic sites** | | | | | | |
| ≤ 2 | 25 (83.3) | 7 (38.9) | <0.01 | 7 (72) | 4 (66.6) | NS |
| > 2 | 5 (16.7) | 11 (61.1) | | 3 (30) | 2 (33.3) | |
| **Pembrolizumab treatment** | | | | | | |
| Monotherapy | 17 (56.7) | 15 (83.3) | NS | 5 (50) | 3 (50.0) | NS |
| Plus chemotherapy | 13 (43.3) | 3 (16.7) | | 5 (50) | 3 (50.0) | |
| **PFS (median days)** | 578 days | 68 days | **<0.001** | 300 days | 80.5 days | **<0.001** |
| **OS (median days)** | 1070 days | 102 days | **<0.001** | NA | 118 days | |

### Example 2.2. Global proteomic analysis of NSCLC blood samples

One hundred thirty-four samples from 48 NSCLC patients were analysed using high-resolution mass spectrometry to identify as many proteins as possible into the plasma samples. First, to obtain quantitative protein information from SWATH-MS, we generated an extensive spectral, based on pooled samples and the online Human Pan-Human library. Thus, our final spectral library, called NSCLibrary, contained assays representing 7,115 unique proteins. Next, we analysed each separated sample against our spectral NSCLibrary and we extracted quantitative data from the set of 18 non-responder and 30 responder patients. An average of 6,483 proteins/sample using a minimum often peptides/protein and a 1% false discovery rate (FDR) on the peptide and protein level were found. Next, after sample normalization by quantile method and sample filtering, we quantified a total of 6,364 unique proteins, of which 2,418 had observations in all samples. In a second step, 37 plasma samples from 16 NSCLC patients corresponding to validation cohort, were also analysed using the same protocol. There were no differences in the proteins' coverage between the responder and non-responder group, neither between the discovery and validation cohorts nor between different time points.

### Example 2.3. Protein plasma levels are associated with immunotherapy response in NSCLC patients

Aiming to identify a protein signature associated with response to immunotherapy, we compared the plasma protein expression of 48 metastatic NSCLC patients prior to pembrolizumab treatment and performed SWATH MS-MS proteomic analyses. Our cohort was divided into two groups of responders (n=30) and non-responders (n=18). We applied Student's t tests with a nominal p-value cut-off of 0.05 (p-value< 0.05) and we identified 324 differentially-expressed proteins (DEPs). Among them, 172 proteins were upregulated, and 152 proteins were downregulated in the responder group, compared to non-responders (**Figure 2**).

Next, we conducted functional enrichment analyses to identify the biological functions and relevant pathways involving into the response to pembrolizumab therapy considered the 324 DEPs found between responder and non-responder patients. Proteomaps to cluster the differentially expressed proteins according to their Genes and Genomes (KEGG) pathway annotations (Figure 3A-B), reported discrete differences between both groups. Despite the results should be taken with caution, responders seem to be dominated by higher levels of metabolic proteins than non-responder patients. The discrete associations found in the analysis can be related with the small number of DEPs between non-responder and responder patients (324 DEPs).

### Example 2.4. Identification and development of a model that allows to predict pembrolizumab response

In order to develop a model that allows us to predict immunotherapy response and take into account DEPs with a tight p-value (p-value <0.01), the inventors selected 66 proteins that were differentially expressed between responder and non-responder patients. Thirty-six of these proteins were upregulated in the responder group, while 30 proteins were upregulated in the non-responder group. These 66 DEPs performed a good discrimination between both groups (**Figure 4**).

However, aiming to obtain a proteome signature with a minimum number of proteins that enables the discrimination between responder and non-responder patients, we used a stepwise method to select a minimum set of proteins associated with response to pembrolizumab therapy (we used p-value <0.01 as the as the criterion: 66 proteins) and created a model of pembrolizumab response in advanced NSCLC patients. This led to the identification of fourteen proteins (SPTN2, PGTA, FIL1L, ATG9A, LZTL1, HPS5, DCDC2, PGAM5, UBAC1, PK3CB, UBP42, ANK1, RS12 and/or EIF3L) that enabled the discrimination between responders and non-responders, yielding areas under the ROC curves (AUCs) of 0.72-0.79 (**Figure 5A**). Among them, eight displayed a significantly higher expression in the non-responder group and six in the responder group (**Figure 5B**).

Further analyses revealed that several combinations of the selected proteins displayed unexpectedly higher AUCs than those provided by any of the individual biomarkers alone. Among them, over 6000 combinations were associated to AUCs above 0.8, some of which are represented in **Table 1.** For instance, combination NO: 1 (consisting of proteins SPTN2, PGTA, FIL1L, ATG9A, LZTL1, HPS5 and DCDC2) and combination NO: 2 (consisting of proteins PGAM5, UBAC1, PK3CB, UBP42, ANK1, RS12 and EIF3L) showed an AUC = 1, evidencing their improved efficacy to discriminate between responder and non-responders (**Table 1**). In addition, multiple combinations of only two of these fourteen proteins yielded unexpectedly high prediction values, such as combination NO: 112 (consisting of proteins FILIL and ATG9A, AUC = 0.915), combination NO: 126 (consisting of proteins RS12 and PGAM5, AUC = 0.906), or combination NO: 129 (consisting of proteins PGAM5 and EIF3L, AUC = 0.904).

Next, an internal validation with an additional cohort of 16 NSCLC patients was performed. Protein levels of the selected proteins was determined in responders (n=10) and non-responders (n=6). Despite the small size of the cohort, all selected proteins showed good AUC in ROCs analyses (AUC= 0.467-0.750) (**Figure 6A**). Importantly, ROC analyses of combination NO: 1 and combination NO: 2 presented excellent discrimination rates in the validation (AUC=1 in both cases) (**Figure 6B**).

### Example 2.5. Possible example of proteome model associated with progression free survival and overall survival in NSCLC patients.

To further validate the clinical significance of the fourteen selected proteins, the inventors investigated their expression levels in association with progression-free survival (PFS) and overall survival (OS) in our global cohort. Kaplan-Meier curve analysis showed that low expression levels of ATG9A were significantly associated with longer PFS (log-rank p < 0.001) in NSCLC patients. On the other hand, high levels of SPTN2 were associated with longer PFS (log-rank p = 0.017). Additionally, low expression levels of HPS5 showed a trend towards being associated with longer PFS (log-rank p = 0.054) and were significantly associated with longer OS (log-rank p < 0.001). Low expression levels of DCDC2 were also significantly associated with longer OS in our global cohort (log-rank p < 0.01) (**Figure 7**).

## Claims

1. *In vitro* method for predicting the response of patients suffering from cancer to a treatment with PD-1 and/or PD-L1 inhibitors, which comprises a) assessing the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, in a biological sample obtained from a subject, b) wherein a higher concentration level of at least one protein selected from the group consisting of: SPTN2, PGTA, UBP42, ANK1, RS12 and/or EIF3L, or a higher expression level of the genes encoding those proteins, or a lower concentration level of at least one protein selected from the group consisting of: FIL1L, ATG9A, LZTL1, HPS5, DCDC2, PGAM5, UBAC1 and/or PK3CB, or lower expression level of the genes encoding those proteins, as compared to a pre-established threshold value, is an indication that the subject will respond to the treatment.

2. *In vitro* method for recommending whether to treat patients suffering from cancer with PD-1 and/or PD-L1 inhibitors, or for classifying patients suffering from cancer into responders or non-responders to a treatment with PD-1 and/or PD-L1 inhibitors, which comprises a) assessing the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, in a biological sample obtained from a subject, b) wherein a higher concentration level of at least one protein selected from the group consisting of: SPTN2, PGTA, UBP42, ANK1, RS12 and/or EIF3L, or a higher expression level of the genes encoding those proteins, or a lower concentration level of at least one protein selected from the group consisting of: FIL1L, ATG9A, LZTL1, HPS5, DCDC2, PGAM5, UBAC1 and/or PK3CB, or a lower expression level of the genes encoding those proteins, as compared to a pre-established threshold value, is an indication that the treatment with PD-1 and/or PD-L1 inhibitors is recommended or that the subject may be classified as a responder to the treatment with PD-1 and/or PD-L1 inhibitors.

3. *In vitro* method, according to any of the previous claims, which comprises a) assessing the concentration level of a combination from two to fourteen proteins selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the subject may be classified as a responder to the treatment with PD-1 and/or PD-L1 inhibitors.

4. *In vitro* method, according to any of the previous claims, which comprises a) assessing the concentration level of at least one of the combinations of proteins of Table 1, or the expression level of the genes encoding those proteins, in a biological sample obtained from a subject, b) processing the concentration or expression levels in order to obtain a score value, and c) wherein if a higher score value is obtained, as compared to a pre-stablished threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

5. *In vitro* method, according to any of the previous claims, which comprises a) assessing the concentration level of the combination of the following proteins: SPTN2, PGTA, FIL1L, ATG9A, LZTL1, HPS5 and DCDC2, or the expression level of the genes encoding those proteins, or the combination of the following proteins: PGAM5, UBAC1, PK3CB, UBP42, ANK1, RS12 and EIF3L, or the expression level of the genes encoding those proteins, in a biological sample obtained from a subject, b) processing the concentration or expression levels in order to obtain a score value, and c) wherein a higher score value is obtained, as compared to a pre-established threshold score, is an indication that the subject will respond to a treatment with PD-1 and/or PD-L1 inhibitors, that treatment with PD-1 and/or PD-L1 inhibitors is recommended, or that the patient may be classified as a responder to the treatment with PD-1 and/or PD-L1 inhibitors.

6. *In vitro* method, according to any of the previous claims, wherein the biological sample obtained from a subject is blood, serum or plasma.

7. *In vitro* method, according to any of the previous claims, wherein the cancer is lung cancer, preferably non-small cell lung cancer (NSCLC).

8. *In vitro* method, according to any of the previous claims, wherein the PD-1 and/or PD-L1 inhibitor is an antibody, preferably an antibody selected from the list comprising: pembrolizumab, nivolumab, atezolizumab, avelumab, durvalumab, cemiplimab or dostarlimab.

9. *In vitro* use of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the genes encoding those proteins, or of a kit consisting of reagents for measuring the concentration level of at least one protein selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or the expression level of the genes encoding those proteins, for predicting the response of patients suffering from cancer to a treatment with PD-1 and/or PD-L1 inhibitors, for recommending whether to treat patients suffering from cancer with PD-1 and/or PD-L1 inhibitors, or for classifying patients suffering from cancer into responders or non-responders to a treatment with PD-1 and/or PD-L1 inhibitors.

10. *In vitro* use, according to claim 9, of a combination from two to fourteen proteins selected from the group consisting of: SPTN2, PGTA, FIL1L, ATG9A, LZTL1, HPS5, DCDC2, PGAM5, UBAC1, PK3CB, UBP42, ANK1, RS12 and/or EIF3L, or of at least one of the combinations of proteins of Table 1, or the combination of the following proteins: SPTN2, PGTA, FIL1L, ATG9A, LZTL1, HPS5 and DCDC2; or PGAM5, UBAC1, PK3CB, UBP42, ANK1, RS12 and EIF3L, or of the expression level of genes encoding those proteins, for predicting the response of patients suffering from cancer to a treatment with PD-1 and/or PD-L1 inhibitors, for recommending whether to treat patients suffering from cancer with PD-1 and/or PD-L1 inhibitors, or for classifying patients suffering from cancer into responders or non-responders to a treatment with PD-1 and/or PD-L1 inhibitors.

11. *In vitro* use, according to any of the claims 9 or 10, wherein the biological sample obtained from a subject is blood, serum or plasma.

12. *In vitro* use, according to any of the claims 9 to 11, wherein the cancer is lung cancer, preferably non-small cell lung cancer (NSCLC).

13. *In vitro* use, according to any of the claims 9 to 12, wherein the PD-1 and/or PD-L1 inhibitor is an antibody, preferably an antibody specifically binding PD-1 and/or PD-L1 selected from the list comprising: pembrolizumab, nivolumab, atezolizumab, avelumab, durvalumab, cemiplimab or dostarlimab.

14. PD-1 and/or PD-L1 inhibitor, or any pharmaceutical composition comprising thereof, optionally including pharmaceutically acceptable excipients or carriers, for use in the treatment of patients suffering from cancer, wherein the method comprises assessing whether the patient is a responder to the treatment by following the method of claims 1 to 8.

15. PD-1 and/or PD-L1 inhibitor for use, according to claim 14, wherein the PD-1 and/or PD-L1 inhibitor is an antibody specifically binding PD-1 and/or PD-L1, preferably an antibody selected from the list comprising: pembrolizumab, nivolumab, atezolizumab, avelumab, durvalumab, cemiplimab or dostarlimab.
